# EUROPEAN PATENT SPECIFICATION

(11) **EP 3 153 509 B1**
(45) Date of publication and mention of the grant of the patent: **15.05.2019**
(21) Application number: 15803726.7
(22) Date of filing: 06.01.2015
(51) Int. Cl.: C07D 401/12

(54) **N-(2-CHLOROMETHYL-1-METHYL-1H-BENZIMIDAZOLE-5-ACYL)-N-(PYRIDINE-2-YL)-3-AMINOPROPANOIC ACID ETHYL ESTER PREPARATION METHOD**
HERSTELLUNGSVERFAHREN FÜR N-(2-CHLORMETHYL-1-METHYL-1H-BENZIMIDAZOL-5-ACYL)-N-(PYRIDIN-2-YL)-3-AMINOPROPANSÄUREETHYLESTER
PROCÉDÉ DE PRÉPARATION DE L'ESTER ÉTHYLIQUE DE L'ACIDE N-(2-CHLOROMÉTHYL-1-MÉTHYL-1H-BENZIMIDAZOLE -5-ACYL)-N-(PYRIDIN-2-YL)-3-AMINOPROPANOÏQUE

(30) Priority: 05.06.2014 CN 201410245997
(43) Date of publication of application: 12.04.2017
(73) Proprietor: ABA Chemicals Corporation, Taicang City, Jiangsu 215433 (CN)
(72) Inventor: LIN, Zhigang, Taicang Jiangsu 215433 (CN); LIU, Yanqin, Taicang Jiangsu 215433 (CN); XU, Jun, Taicang Jiangsu 215433 (CN); JIANG, Yueheng, Taicang Jiangsu 215433 (CN); CAI, Tong, Taicang Jiangsu 215433 (CN)
(74) Representative: Proi World Intellectual Property GmbH
(86) International application number: PCT/CN2015/070175
(87) International publication number: WO 2015/184797

(56) References cited:
- CN-A- 1 248 251
- CN-A- 102 612 517
- CN-A- 102 633 713
- CN-A- 102 850 325

## Description

### Technical Field

The Invention relates to a method for preparing N-(2-chloromethyl- 1-methyl-1H-benzimidazole-5-acyl)-N-(pyridine-2-yl)-3-aminopropionic acid ethyl ester (a Dabigatran etexilate intermediate).

### Background Art

N-(2-chloromethyl-1-methyl-1H-benzimidazole-5-acyl)-N-(pyridine-2-yl)-3-am inopropionic acid ethyl ester is an important intermediate of Dabigatran Etexilate, a new anticoagulant developed and listed by Boehringer Ingelheim Company of Germany. We have synthesized this intermediate through the synthetic method provided by its published reports (WO2011061080, etc.) and found that the solubility of N-(pyridine-2-yl)-N-(4-methylamino-3-nitrobenzene formyl)- 3- aminopropionic acid ethyl ester is a major problem, resulting in a large amount of solvent for hydrogenation reaction with low recovery rate and low purity.

### Summary of the Invention

The technical problem to be solved by the Invention is to provide a method for preparing N-(2-chloromethyl-1-methyl-1H-benzimidazole-5-acyl)-N-(pyridine-2-yl)-3-aminopropionic acid ethyl ester (a Dabigatran etexilate intermediate).

**The technical scheme adopted by the Invention to solve the technical problem is as follows:**
A method for preparing N-(2-chloromethyl-1-methyl-1H-benzimidazole-5 -acyl)- N- (pyridine- 2-yl)- 3-aminopropionic acid ethyl ester, wherein, it comprises the following steps:
(1) Taking 3-amino-4-methylamino benzoic acid as the raw material, synthesizing 2-chloromethyl-1-methyl-1H-benzimidazole-5-carboxylic acid through cyclization reaction with chloroacetyl chloride;
(2) The said 2-chloromethyl-1-methyl-1H-benzimidazole-5-carboxylic acid generates 2-chloromethyl-1-methyl-1H-benzimidazole-5 formyl chloride through chlorination reaction under the action of acyl chloride;
(3) The said 2-chloromethyl-1-methyl-1H-benzimidazole-5-formyl chloride generates the said N-(2-chloromethyl-1-methyl-1H-benzimidazole-5-acyl)-N-(pyridine-2-yl)- 3- aminopropionic acid ethyl ester through condensation reaction with 3-(pyridine-2-yl) aminopropionic acid ethyl ester.

Preferably, in Step (1), the mole ratio between chloroacetyl chloride and the raw material 3-amino-4-methylamino benzoic acid is (0.9-2):1.

Preferably, in Step (1), the said cyclization reaction is occurred in a chloroacetyl chloride/organic solvent system; the said organic solvent is one or more of the solvents as ethyl acetate, acetonitrile, DMF or formamide.

Preferably, in Step (1), the temperature for the said cyclization reaction is 50-150°C and the time is 1-24 hours; the preferred reaction temperature is 50-70°C and the time is 1-3 hours.

Preferably, in Step (2), the said 2-chloromethyl-1-methyl-1H-benzimidazole-5-carboxylic acid generates 2-chloromethyl-1-methyl-1H-benzimidazole-5 formyl chloride in a chloride/organic solvent system through chlorination reaction; the said organic solvent is one or more of the solvents as dichloromethane, dichloroethane, ethyl acetate and acetonitrile.

Preferably, in Step (2), the said acyl chloride is oxalyl chloride, thionyl chloride or chloro-carbonic ester.

Preferably, in Step (2), the temperature for the said chlorination reaction is 0-100°C and the time is 0.5-24 hour; the preferred reaction temperature is 15-20°C and the time is 0.5-2 hours.

Preferably, in Step (3), the mole ratio between the said 2-chloromethyl-1-methyl-1H-benzimidazole-5-formyl chloride and the raw material 3-(pyridine-2-yl) aminopropionic acid ethyl ester is (0.9-2):1.

Preferably, in Step (3), the said condensation reaction is occurred in an organic solvent system; the said organic solvent is one or more of the solvents as ethyl acetate, acetonitrile, DMF or formamide.

Preferably, in Step (3), the temperature for the said condensation reaction is 0-80°C and the time is 1-24 hour; the preferred reaction temperature is 15-20°C and the time is 1-2 hours.

The above preparation method is expressed by a chemical equation as follows:

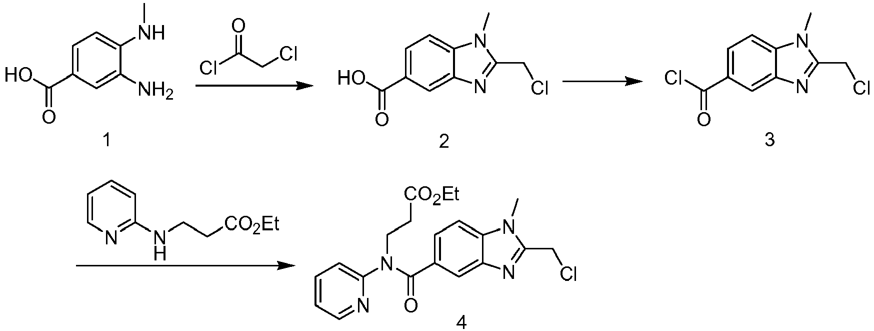

The beneficial effects of the Invention are that: the synthetic route has not been reported ever before; moreover, with cheap and widely available raw materials, simple unit operation and low requirement for equipment, the method is suitable for industrialized mass production.

The partial additional aspects and advantages of the Invention are given as follows, some of which will be obvious through the following description, or be understood in practice of the Invention.

### Detailed Description of the Preferred Embodiments

The following detailed embodiments are further descriptions of the technical schemes of the Invention, but the Invention is not limited to this.

### (1) Synthesis of 2-chloromethyl-1-methyl-1H-benzimidazole-5-carboxylic acid (I)

Add 61.2g of 3-amino-4-methylamino benzoic acid hydrochloride and 400ml of DMF into a 1000 mL reaction bulb; slowly raise the temperature to 50°C and add 30ml of chloroacetyl chloride by drops to react for 2 hours; control the raw material below 0.2% through HPLC monitoring; add 400ml of ethyl acetate (the reactant is dissolved in DMF; adding ethyl acetate is to precipitate the product) and cool it to 0°C and filter it to obtain a raw product of 2-chloromethyl-1-methyl-1H-benzimidazole-5-carboxylic acid hydrochloride. Dissolve the raw product into a mixed solvent of 400ml of water and 400ml of acetonitrile; adjust the pH value to 4-5 with 3N aqueous sodium hydroxide and precipitate the solid; filter it and wash it with the mixed solvent of water and acetonitrile (with a volume proportion of 1/3, which is not fixed and can be changed) and dry it to obtain the product 2-chloromethyl-1-methyl-1H-benzimidazole-5-carboxylic acid of 58 g; calculated yield: 85.5%; purity: 99.21%; LC-MS (m/e): 224.04.

### Synthesis of 2-chloromethyl-1-methyl-1H-benzimidazole-5-carboxylic acid (II)

Add 61.2g of 3-amino-4-methylamino benzoic acid hydrochloride, 400ml of DMF and 200ml of acetonitrile into a 1000 mL reaction bulb; slowly raise the temperature to 50°C for reaction; then add 30ml of chloroacetyl chloride by drops to react for 2 hours and control the raw material below 0.2% through HPLC monitoring; add 400ml of acetonitrile (a low-polarity solvent, with which the product can be precipitated) and cool it to 0°C and filter it to obtain a raw product of 2-chloromethyl-1-methyl-1H-benzimidazole-5-carboxylic acid hydrochloride. Dissolve the raw product into a mixed solvent of 400ml of water and 400ml of acetonitrile; adjust the pH value to 4-5 with 3N aqueous sodium hydroxide and dissolve out the solid; filter it and wash it with the mixed solvent of water and acetonitrile (with a volume proportion of 1/3, which is not fixed and can be changed) and dry it to obtain the product 2-chloromethyl-1-methyl-1H-benzimidazole-5-carboxylic acid of 60 g; calculated yield: 87.7%; purity: 99.29%; LC-MS (m/e): 224.04.

### (2) Synthesis of 2-chloromethyl-1-methyl-1H-benzimidazole-5 formyl chloride (I)

Add 49g of 2-chloromethyl-1-methyl-1H-benzimidazole-5-carboxylic acid synthetized through Step (1) (I), 750ml of dichloromethane and catalytic amounts of DMF into a 1000 mL reaction bulb and control the temperature within 15-20°C; then add 22ml of oxalyl chloride by drops to react under ambient temperature for 1 hour; reduce the pressure to concentrate the volume to 1/2; then add 500ml of ethyl acetate (ethyl acetate can take away dichloromethane and redundant oxalyl chloride) and reduce the pressure to concentrate the volume to 1/2 to obtain an ethyl acetate solution of 2-chloromethyl-1-methyl-1H-benzimidazole-5 formyl chloride; it is calculated with 100% yield and directly used in the next reaction.

### Synthesis of 2-chloromethyl-1-methyl-1H-benzimidazole-5 formyl chloride (II)

Add 49g of 2-chloromethyl-1-methyl-1H-benzimidazole-5-carboxylic acid synthetized through Step (1) (II), 750ml of dichloroethane and catalytic amounts of DMF into a 1000 mL reaction bulb and control the temperature within 15-20°C; then add 24ml of thionyl chloride by drops to react under ambient temperature for 1 hour; reduce the pressure to concentrate the volume to 1/2; then add 500ml of ethyl acetate and reduce the pressure to concentrate the volume to 1/2 again to obtain an ethyl acetate solution of 2-chloromethyl-1-methyl-1H-benzimidazole-5 formyl chloride; it is calculated with 100% yield and directly used in the next reaction.

### (3) Synthesis of N-(2-chloromethyl-1-methyl-1H-benzimidazole-5-acyl)-N -(pyridine-2-yl)-3-aminopropionic acid ethyl ester (I)

Add 39g of 3-(pyridine-2-yl) aminopropionic acid ethyl ester synthetized through Step (2) (I), 200ml of ethyl acetate and 56ml of triethylamine into a 1000 mL reaction bulb; control the temperature within 15-20°C; then add the ethyl acetate solution of 2-chloromethyl-1-methyl-1H-benzimidazole-5 formyl chloride by drops for stirring under ambient temperature for 1 hour; control the raw material below 0.2% through HPLC monitoring; then terminate the reaction. Add 500ml of water for quenching the reaction to separate out organic phase; extract it with ethyl acetate; wash it with water; dry it with anhydrous sodium sulfate, filter and concentrate it to obtain the raw product; then add the mixed solvent of ethyl acetate and cyclohexane for recrystallization and obtain a product of 68g. Calculated yield: 85%; purity: 99.51%; LC-MS (m/e): 400.1.

### Synthesis of N-(2-chloromethyl-1-methyl-1H-benzimidazole-5-acyl)-N -(pyridine-2-yl)-3-aminopropionic acid ethyl ester (II)

Add 39g of 3-(pyridine-2-yl) aminopropionic acid ethyl ester synthetized through Step (2) (II), 200ml of acetonitrile and 56ml of triethylamine into a 1000 mL reaction bulb; control the temperature within 15-20°C; then add the ethyl acetate solution of 2-chloromethyl-1-methyl-1H-benzimidazole-5 formyl chloride by drops for stirring under ambient temperature for 1 hour; control the raw material below 0.2% through HPLC monitoring; then terminate the reaction. Add 500ml of water for quenching the reaction to separate out organic phase; extract it with ethyl acetate; wash it with water; dry it with anhydrous sodium sulfate, filter and concentrate it to obtain the raw product; then add the mixed solvent of ethyl acetate and cyclohexane for recrystallization and obtain a product of 66g. Calculated yield: 82.5%; purity: 99.61%; LC-MS (m/e): 400.1.

## Claims

1. A method for preparing N-(2-chloromethyl-1-methyl-1H-benzimidazole-5-acyl)-N-(pyridine-2-yl)-3-aminopropionic acid ethyl ester, wherein, it comprises the following steps:
(1) taking 3-amino-4-methylamino benzoic acid as the raw material, synthesizing 2-chloromethyl-1-methyl-1H-benzimidazole-5-carboxylic acid through cyclization reaction with chloroacetyl chloride;
(2) said 2-chloromethyl -1-methyl-1H-benzimidazole-5-carboxylic acid generates 2-chloromethyl -1-methyl-1H-benzimidazole-5 formyl chloride through chlorination reaction under the action of acyl chloride;
(3) said 2-chloromethyl -1-methyl-1H-benzimidazole-5 formyl chloride generates the said N-(2-chloromethyl-1-methyl-1H-benzimidazole- 5-acyl)-N-(pyridine-2-yl)-3-aminopropionic acid ethyl ester through condensation reaction with 3-(pyridine-2-yl) aminopropionic acid ethyl ester.

2. The preparation method according to Claim 1, wherein, in Step (1), the mole ratio between chloroacetyl chloride and the raw material 3-amino-4-methylamino benzoic acid is (0.9-2):1.

3. The preparation method according to Claim 1, wherein, in Step (1), the said cyclization reaction is occurred in a chloroacetyl chloride/organic solvent system; the said organic solvent is one or more of the solvents as ethyl acetate, acetonitrile, DMF or formamide.

4. The preparation method according to Claim 1, wherein, in Step (1), the temperature for the said cyclization reaction is 50-150°C and the time is 1-24 hours; the preferred reaction temperature is 50-70°C and the time is 1-3 hours.

5. The preparation method according to Claim 1, wherein, in Step (2), the said 2-chloromethyl-1-methyl-1H-benzimidazole-5-carboxylic acid generates 2-chloromethyl-1-methyl-1H-benzimidazole-5 formyl chloride in a chloride/organic solvent system through chlorination reaction; the said organic solvent is one or more of the solvents as dichloromethane, dichloroethane, ethyl acetate and acetonitrile.

6. The preparation method according to Claim 1 or 5, wherein, in Step (2), the said acyl chloride is oxalyl chloride, thionyl chloride or chloro-carbonic ester.

7. The preparation method according to Claim 1, wherein, in Step (2), the temperature for the said chlorination reaction is 0-100°C and the time is 0.5-24 hours; the preferred reaction temperature is 15-20°C and the time is 0.5-2 hours.

8. The preparation method according to Claim 1, wherein, in Step (3), the mole ratio between the said 2-chloromethyl -1-methyl-1H-benzimidazole-5-formyl chloride and the raw material 3-(pyridine-2-yl) aminopropionic acid ethyl ester is (0.9-2):1.

9. The preparation method according to Claim 1, wherein, in Step (3), the said condensation reaction is occurred in an organic solvent system; the said organic solvent is one or more of the solvents as ethyl acetate, acetonitrile, DMF or formamide.

10. The preparation method according to Claim 1, wherein, in Step (3), the temperature for the said condensation reaction is 0-80°C and the time is 1-24 hours; the preferred reaction temperature is 15-20°C and the time is 1-2 hours.

## Patentansprüche

1. Verfahren zur Herstellung von N-(2-Methylchlorid-1-Methyl-1H-Benzimidazol-5-Acetyl)-N-(Pyridin-2-yl)-3-Aminopropionsäureethylester, wobei das Verfahren die folgenden Schritte umfasst:
(1) 3-Amino-4-Methylaminobenzoesäure wird als Ausgangsmaterial genommen, Synthese von 2-Methylchlorid-1-Methyl-1H-Benzimidazol-5-Karbonsäure durch Cyclisierungsreaktion mit Chloracetylchlorid;
(2) die 2-Methylchlorid-1-Methyl-1H-Benzimidazol-5-Karbonsäure erzeugt 2-Methylchlorid-1-Methyl-1H-Benzimidazol-5-Formylchlorid durch Chlorierungsreaktion unter Einwirkung von Acylchlorid;
(3) die 2-Methylchlorid-1-Methyl-1H-Benzimidazol-5-Karbonsäure erzeugt das N-(2-Methylchlorid-1-Methyl-1H-Benzimidazol-5-Acyl)-N-(Pyridin-2-yl)-3-Aminopropionsäureethylester durch Kondensationsreaktion mit 3-(Pyridin-2-yl) Aminopropionsäureethylester.

2. Herstellungsverfahren nach Anspruch 1, wobei in Schritt (1) das Molverhältnis zwischen Chloracetylchlorid und dem Rohmaterial 3-Amino-4-Methylaminobenzoesäure (0,9-2):1 beträgt.

3. Herstellungsverfahren nach Anspruch 1, wobei in Schritt (1) die Cyclisierungsreaktion in einem Chloracetylchlorid/organischen Lösungsmittelsystem erfolgt; das organische Lösungsmittel ist eines oder mehrere der Lösungsmittel wie Ethylacetat, Acetonitril, DMF oder Formamid.

4. Herstellungsverfahren nach Anspruch 1, wobei in Schritt (1) die Temperatur für die Cyclisierungsreaktion 50 bis 150°C beträgt und die Zeit 1 bis 24 Stunden beträgt; die bevorzugte Reaktionstemperatur beträgt 50 bis 70°C und die Zeit beträgt 1 bis 3 Stunden.

5. Herstellungsverfahren nach Anspruch 1, wobei in Stufe (2) die 2-Methylchlorid-1-Methyl-1H-Benzimidazol-5-Karbonsäure 2-Methylchlorid-1-Methyl-1H-Benzimidazol-5 erzeugt Formylchlorid in einem Chlorid/organischen Lösungsmittelsystem mittels Chlorierungsreaktion; das organische Lösungsmittel ist eines oder mehrere der Lösungsmittel wie Dichlormethan, Dichlorethan, Ethylacetat und Acetonitril.

6. Herstellungsverfahren nach Anspruch 1 oder 5, wobei das Acylchlorid in Schritt (2) Oxalylchlorid, Thionylchlorid oder Chlorkohlensäureester ist.

7. Herstellungsverfahren nach Anspruch 1, wobei in Schritt (2) die Temperatur für die Chlorierungsreaktion 0 bis 100°C beträgt und die Zeit 0,5 bi 24 Stunden beträgt; die bevorzugte Reaktionstemperatur beträgt 15 bis 20°C und die Zeit beträgt 0,5 bis 2 Stunden.

8. Herstellungsverfahren nach Anspruch 1, wobei in Schritt (3) das Molverhältnis zwischen dem 2-Methylchlorid-1-Methyl-1H-Benzimidazol-5-Formylchlorid und dem Ausgangsmaterial 3- (Pyridin-2-yl) Aminopropionsäureethylester (0,9-2):1 beträgt.

9. Herstellungsverfahren nach Anspruch 1, wobei in Schritt (3) die Kondensationsreaktion in einem organischen Lösungsmittelsystem erfolgt; das organische Lösungsmittel ist eines oder mehrere der Lösungsmittel wie Ethylacetat, Acetonitril, DMF oder Formamid.

10. Herstellungsverfahren nach Anspruch 1, wobei in Schritt (3) die Temperatur für die Kondensationsreaktion 0 bis 80°C beträgt und die Zeit 1 bis 24 Stunden beträgt; die bevorzugte Reaktionstemperatur beträgt 15 bis 20°C und die Zeit beträgt 1 bis 2 Stunden.

## Revendications

1. Procédé destiné à la préparation de l'ester éthylique d'acide N-(2-chlorométhyl-1-méthyl-1H-benzimidazole-5-acyl)-N-(pyridine-2-yl)-3-aminopropionique, le procédé comprenant les étapes suivantes :
(1) la prise d'acide 3-amino-4-méthylamino benzoïque comme la matière première, la synthèse de l'acide 2-chlorométhyl-1-méthyl-1H-benzimidazole-5-carboxylique au moyen de la réaction de cyclisation avec le chlorure de chloroacétyle ;
(2) ledit acide 2-chlorométhyl-1-méthyl-1H-benzimidazole-5-carboxylique génère le chlorure de formyle 2-chlorométhyl-1-méthyl-1H-benzimidazole-5 au moyen de la réaction de chloration sous l'action du chlorure d'acyle ;
(3) ledit chlorure de formyle 2-chlorométhyl-1-méthyl-1H-benzimidazole-5 génère ledit ester éthylique d'acide N-(2-chlorométhyl-1-méthyl-1H-benzimidazole-5-acyl)-N-(pyridine-2-yl)-3-aminopropionique au moyen de la réaction de condensation avec l'ester éthylique d'acide 3-(pyridine-2-yl)-aminopropionique.

2. Procédé de préparation selon la revendication 1, dans lequel, dans l'étape (1), le rapport molaire entre le chlorure de chloroacétyle et la matière première acide 3-amino-4-(méthylamino)benzoïque est (0,9-2):1.

3. Procédé de préparation selon la revendication 1, dans lequel, dans l'étape (1), la réaction de cyclisation est survenue dans un système de solvants chlorure de chloroacétyle/organiques ; ledit solvant organique est l'un ou plusieurs parmi les solvants comme l'acétate d'éthyle, l'acétonitrile, le DMF ou le formamide.

4. Procédé de préparation selon la revendication 1, dans lequel, dans l'étape (1), la température destinée à ladite réaction de cyclisation est de 50 à 150 °C et le temps est de 1 à 24 heures ; la température de réaction préférée est de 50 à 70 °C et le temps est de 1 à 3 heures.

5. Procédé de préparation selon la revendication 1, dans lequel, dans l'étape (2), ledit acide 2-chlorométhyl-1-méthyl-1H-benzimidazole-5-carboxylique génère le chlorure de formyle 2-chlorométhyl-1-méthyl-1H-benzimidazole-5 dans un système de solvants chlorure/organiques au moyen de la réaction de chloration ; ledit solvant organique est l'un ou plusieurs parmi les solvants comme le dichlorométhane, le dichloroéthane, l'acétate d'éthyle et l'acétonitrile.

6. Procédé de préparation selon la revendication 1 ou 5, dans lequel, dans l'étape (2), ledit chlorure d'acyle est le chlorure d'oxalyle, le chlorure de thionyle ou l'ester chlorocarbonique.

7. Procédé de préparation selon la revendication 1, dans lequel, dans l'étape (2), la température destinée à ladite réaction de chloration est de 0 à 100 °C et le temps est de 0,5 à 24 heures ; la température de réaction préférée est de 15 à 20 °C et le temps est de 0,5 à 2 heures.

8. Procédé de préparation selon la revendication 1, dans lequel, dans l'étape (3), le rapport molaire entre ledit chlorure de formyle 2-chlorométhyl-1-méthyl-1H-benzimidazole-5 et la matière première ester éthylique d'acide 3-(pyridine-2-yl)-aminopropionique est (0,9-2):1.

9. Procédé de préparation selon la revendication 1, dans lequel, dans l'étape (3), ladite réaction de condensation est survenue dans un système de solvants organiques ; ledit solvant organique est l'un ou plusieurs parmi les solvants comme l'acétate d'éthyle, l'acétonitrile, le DMF ou le formamide.

10. Procédé de préparation selon la revendication 1, dans lequel, dans l'étape (3), la température destinée à ladite réaction de condensation est de 0 à 80 °C et le temps est de 1 à 24 heures ; la température de réaction préférée est de 15 à 20 °C et le temps est de 1 à 2 heures.
